(19) Europäisches Patentamt
European Patent Office
Office européen des brevets

(11) Veröffentlichungsnummer: **0 070 464 B1**

# (12) EUROPÄISCHE PATENTSCHRIFT

(45) Veröffentlichungstag der Patentschrift: **02.10.85**

(51) Int. Cl.⁴: **C 07 C 69/74**, C 07 C 121/75, C 07 C 149/40, C 07 D 213/64, C 07 C 67/08, A 01 N 53/00

(21) Anmeldenummer: **82106113.2**

(22) Anmeldetag: **08.07.82**

(54) **Substituierte Phenyl-cyclopropancarbonsäureester, Verfahren zu ihrer Herstellung und ihre Verwendung als Schädlingsbekämpfungs-mittel.**

(30) Priorität: **18.07.81 DE 3128444**

(43) Veröffentlichungstag der Anmeldung: **26.01.83 Patentblatt 83/4**

(45) Bekanntmachung des Hinweises auf die Patenterteilung: **02.10.85 Patentblatt 85/40**

(84) Benannte Vertragsstaaten: **AT BE CH DE FR GB IT LI NL**

(56) Entgegenhaltungen:
**EP - A - 0 017 952**
**EP - A - 0 019 787**
**EP - A - 0 043 492**
**DE - A - 2 837 524**
**DE - A - 2 855 422**
**DE - A - 2 949 342**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Fuchs, Rainer, Dr., Roeberstrasse 8, D-5600 Wuppertal 1 (DE)**
Erfinder: **Klauke, Erich, Dr., Eichendorffweg 8, D-5063 Odenthal (DE)**
Erfinder: **Hammann, Ingeborg, Dr., Belfortstrasse 9, D-5000 Koeln (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 070 464 B1

**Beschreibung**

Die Erfindung betrifft neue substituierte Phenyl-cyclopropancarbonsäureester, ein Verfahren zu ihrer Herstellung und ihre Verwendung als Schädlingsbekämpfungsmittel sowie neue Zwischenprodukte hierfür und Verfahren zu deren Herstellung.

Es ist bekannt, daß bestimmte Phenyl-cyclopropancarbonsäureester, wie z. B. 3-(4-Methoxy-phenyl)-2,2-dimethyl-cyclopropancarbonsäure-(α-cyano-3-phenoxybenzyl)-ester, insektizid wirksam sind (vgl. DE-OS 2 855 422).

Die Wirkung dieser Verbindungen ist jedoch, insbesondere bei niedrigen Wirkstoffkonzentrationen und Aufwandmengen, nicht immer zufriedenstellend.

Gegenstand der vorliegenden Erfindung sind

1) neue substituierte Phenyl-cyclopropancarbonsäureester der Formel (I)

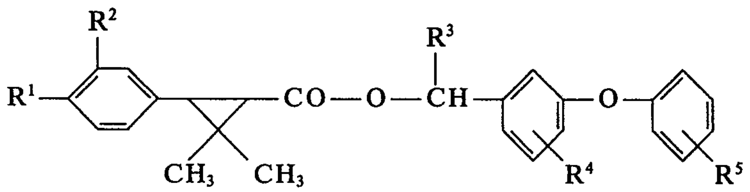

(I)

in welcher

$R^1$ für einfach oder mehrfach, gleich oder verschieden halogensubstituierte Alkoxy- oder Alkylthioreste steht,
$R^2$ für Wasserstoff oder Halogen steht,
$R^1$ und $R^2$ gemeinsam für einfach oder mehrfach gleich oder verschieden halogen-substituiertes Alkylendioxy stehen,
$R^3$ für Wasserstoff oder Cyano steht,
$R^4$ für Halogen steht, und
$R^5$ für Wasserstoff oder Halogen steht;

2) ein Verfahren zur Herstellung der neuen Verbindungen der Formel (I)

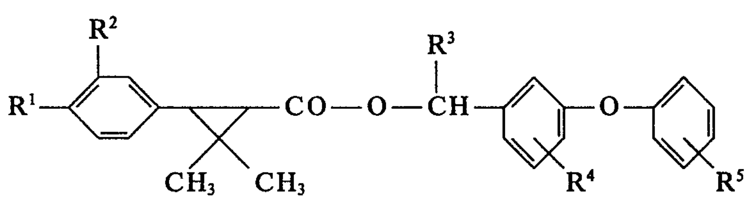

(I)

in welcher

$R^1$ für einfach oder mehrfach, gleich oder verschieden halogensubstituierte Alkoxy- oder Alkylthioreste steht,
$R^2$ für Wasserstoff oder Halogen steht,
$R^1$ und $R^2$ gemeinsam für einfach oder mehrfach, gleich oder verschieden halogen-substituiertes Alkylendioxy stehen,
$R^3$ für Wasserstoff oder Cyano steht,
$R^4$ für Halogen steht, und
$R^5$ für Wasserstoff oder Halogen steht,

dadurch gekennzeichnet, daß man substituierte 2,2-Dimethyl-3-phenyl-cyclopropancarbonsäuren der Formel (II)

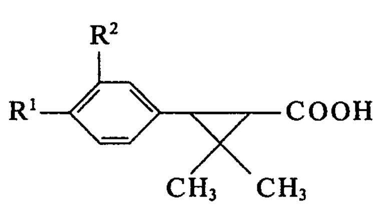

(II)

in welcher

$R^1$ und $R^2$ die oben angegebenen Bedeutungen haben,

— oder reaktionsfähige Derivate derselben, mit 3-Phenoxybenzyl-alkoholen der Formel (III)

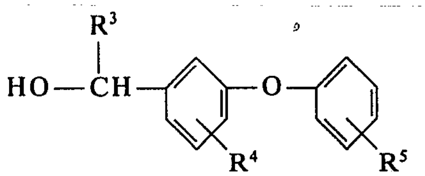

(III)

in welcher

$R^3$, $R^4$, $R^5$ die unter (I) angegebenen Bedeutungen haben,

oder mit deren reaktionsfähigen Derivaten, gegebenenfalls in Gegenwart eines Säureakzeptors und/oder eines Katalysators und gegebenenfalls unter Verwendung eines oder mehrerer Verdünnungsmittel umsetzt:

3) neue substituierte 2,2-Dimethyl-3-phenyl-cyclopropancarbonsäuren der Formel (II)

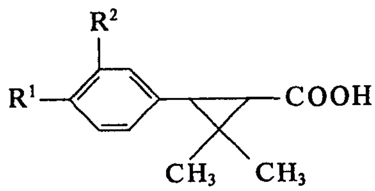

(II)

in welcher

$R^1$ für einfach oder mehrfach, gleich oder verschieden halogensubstituierte $C_1-C_4$-Alkoxy- oder $C_1-C_4$-Alkylthioreste steht,
$R^2$ für Wasserstoff oder Halogen steht oder
$R^1$ und $R^2$ gemeinsam für einfach oder mehrfach, gleich oder verschieden halogen-substituiertes Alkylendioxy stehen;

4) ein Verfahren zur Herstellung der neuen Verbindungen der Formel (II), dadurch gekennzeichnet, daß man substituierte 2,2-Dimethyl-3-phenyl-cyclopropancarbonsäureester der Formel (IV)

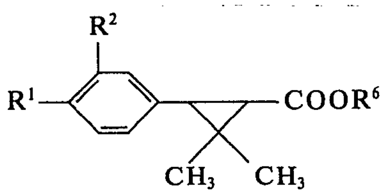

(IV)

in welcher

$R^1$ und $R^2$ die oben angegebenen Bedeutungen haben und
$R^6$ für $C_1-C_4$-Alkyl steht,

mit Alkalihydroxiden in Gegenwart von Verdünnungsmitteln auf Temperaturen zwischen 50 und 200° C erhitzt und anschließend bei Raumtemperatur mit Mineralsäuren ansäuert.

5) Substituierte 2,2-Dimethyl-3-phenyl-cyclopropancarbonsäureester der Formel IV

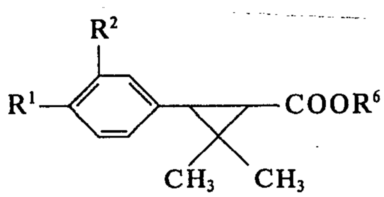

(IV)

in welcher

$R^1$ für einfach oder mehrfach, gleich oder verschieden halogen-substituierte Alkoxy- oder Alkylthioreste steht,

$R^2$ für Wasserstoff oder Halogen steht, oder
$R^1$ und $R^2$ gemeinsam für einfach oder mehrfach, gleich oder verschieden halogen-substituiertes Alkylendioxy stehen und
$R^6$ für $C_1-C_4$-Alkyl steht sind neu. Sie werden erhalten, indem man

6) substituierte 2-Methyl-3-phenyl-propane der Formel V

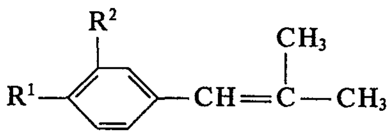

(V)

in welcher

$R^1$ und $R^2$ die oben angegebenen Bedeutungen haben,

mit Diazoessigsäureestern der Formel VI

$N_2CH-COOR^6$ (VI)

in welcher

$R^6$ die unter (4) (oben) angegebene Bedeutung hat,

in Gegenwart eines Katalysators bei Temperaturen zwischen 50 und 200° C umsetzt.

7) Substituierte 2-Methyl-3-phenyl-propene der Formel V

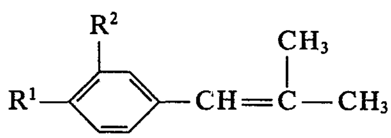

(V)

in welcher

$R^1$ für einfach oder mehrfach, gleich oder verschieden halogensubstituierte Alkoxy- oder Alkylthioreste steht,
$R^2$ für Wasserstoff oder Halogen steht oder
$R^1$ und $R^2$ gemeinsam für einfach oder mehrfach, gleich oder verschieden halogen-substituiertes Alkylendioxy stehen sind neu. Sie werden erhalten, indem man

8) substituierte Benzaldehyde der Formel VII

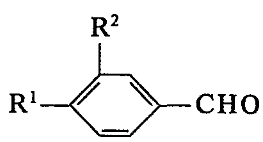

(VII)

in welcher

$R^1$ und $R^2$ die oben angegebenen Bedeutungen haben,

mit Triphenyl-isopropyl-phosphoran der Formel VIII

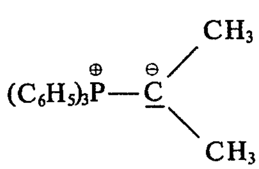

(VIII)

gegebenenfalls unter Verwendung von Verdünnungsmitteln bei Temperaturen zwischen −70 und +50° C umsetzt.

9) Substituierte Benzaldehyde der Formel VII

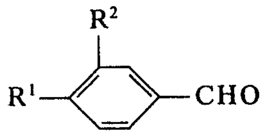

(VII)

in welcher

$R^1$ für einfach oder mehrfach, gleich oder verschieden halogensubstituierte Alkoxy- oder Alkylthioreste steht, und
$R^2$ für Halogen steht sind neu. Sie werden erhalten, indem man

10) substituierte Benzole der Formel IX

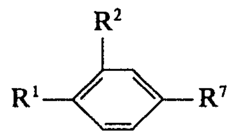

(IX)

in welcher

$R^1$ und $R^2$ die unter (9) angegebenen Bedeutungen haben und
$R^7$ für Hydroxymethyl steht,

in üblicher Weise in die entsprechenden Aldehyde überführt.

Die neuen Verbindungen der Formel (I) zeichnen sich durch hohe insektizide und akarizide Wirksamkeit aus.

Überraschenderweise zeigen die erfindungsgemäßen Verbindungen der Formel (I) eine erheblich höhere insektizide und akarizide Wirkung als aus dem Stand der Technik bekannte Verbindungen analoger Konstitution und gleicher Wirkungsrichtung.

Die allgemeine Formel (I) schließt die verschiedenen möglichen Stereoisomeren und optisch aktiven Isomeren sowie deren Mischungen mit ein.

Gegenstand der Erfindung sind vorzugsweise substituierte Phenyl-cyclopropancarbonsäureester der Formel (I)

in welcher

$R^1$ für $C_1-C_2$-Fluoralkoxy, $C_1-C_2$-Chlorfluoralkoxy, $C_1-C_2$-Fluoralkylthio oder $C_1-C_2$-Chlorfluoralkylthio steht,
$R^2$ für Wasserstoff, Chlor oder Brom steht,
$R^1$ und $R^2$ gemeinsam für $C_1-C_2$-Fluoralkylendioxy stehen,
$R^3$ für Wasserstoff oder Cyano steht,
$R^4$ für Fluor, Chlor oder Brom steht, und
$R^5$ für Wasserstoff, Fluor, Chlor oder Brom steht.

Ganz besonders bevorzugt sind die substituierten Phenyl-cyclopropancarbonsäureester der Formel (I),

in welcher

$R^1$ für Chlortrifluorethoxy, Tetrafluorethoxy, Chlordifluormethoxy, Trifluormethoxy, Trifluormethylthio und Difluormethoxy steht,
$R^2$ für Wasserstoff, Chlor oder Brom steht,
$R^1$ und $R^2$ gemeinsam für Trifluorethylendioxy oder Difluormethylendioxy stehen,
$R^3$ für Wasserstoff oder Cyano steht,
$R^4$ für 4-Fluor, 4-Chlor oder 4-Brom steht,
$R^5$ für Wasserstoff steht.

Im einzelnen seien außer den bei den Herstellungsbeispielen genannten Verbindungen die folgenden Verbindungen der Formel (I) genannt:

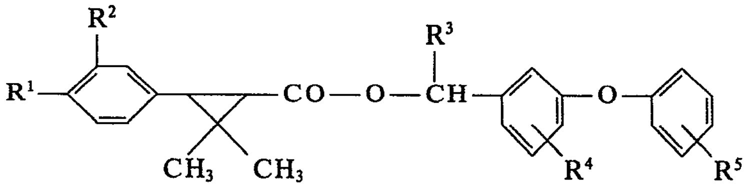

(I)

| $R^1$ | $R^2$ | $R^3$ | $R^4$ | $R^5$ |
|---|---|---|---|---|
| $F_3CO$ | Cl | H | 4-F | H |
| $F_3CO$ | Cl | CN | 4-F | H |
| $F_2HCO$ | H | H | 4-F | H |
| $F_2HCO$ | Cl | CN | 4-F | H |
| $-O-CHF-CF_2-O-$ | | CN | 4-F | H |
| $-O-CHF-CF_2-O-$ | | H | 4-F | H |
| $-O-CF_2-O-$ | | CN | 4-F | H |
| $-O-CF_2-O-$ | | H | 4-F | H |
| $FClHC-CF_2O-$ | H | H | 4-F | H |
| $FClHC-CF_2O-$ | Cl | CN | 4-F | H |
| $F_2HC-CF_2O-$ | Cl | H | 4-F | H |
| $F_2HC-CF_2O-$ | Cl | CN | 4-F | H |

Eine bevorzugte Variante (a) des unter (2) dargelegten Verfahrens zur Herstellung der neuen Verbindungen der Formel (I) — »Verfahren (2a)« — ist dadurch gekennzeichnet, daß man als reaktionsfähige Derivate der substituierten 2,2-Dimethyl-3-phenyl-cyclopropancarbonsäure der Formel (II) substituierte 2,2-Dimethyl-3-phenyl-cyclopropancarbonsäurechloride der Formel IIa

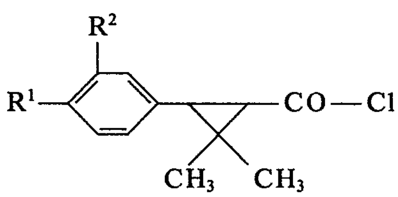

(IIa)

in welcher

$R^1$ und $R^2$ die unter (1) angegebenen Bedeutungen haben,

mit 3-Phenoxy-benzyl- bzw. 6-Phenoxy-2-pyridyl-alkoholen der Formel (III) (oben) in Gegenwart von Säureakzeptoren und unter Verwendung von Verdünnungsmitteln umsetzt.

Eine weitere bevorzugte Variante (b) des unter (2) dargelegten Verfahrens zur Herstellung von Verbindungen der Formel (I), in welcher $R^3$ für Cyano steht — »Verfahren (2b)« — ist dadurch gekennzeichnet, daß man Carbonsäurechloride der Formel IIa (oben) mit 3-Phenoxybenzaldehyden der Formel IIIa

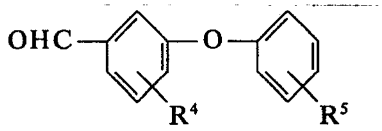

(IIIa)

in welcher

$R^4$, $R^5$ die unter (1) angegebenen Bedeutungen haben,

und wenigstens der äquimolaren Menge eines wasserlöslichen Cyanids (vorzugsweise Natriumcyanid oder Kaliumcyanid), gegebenenfalls in Gegenwart eines Katalysators und unter Verwendung von Verdünnungsmitteln umsetzt.

Verwendet man als Ausgangsstoffe beispielsweise 3-(3-Brom-4-trifluormethoxy-phenyl)-cyclopropancarbonsäurechlorid und 3-Phenoxy-4-fluor-benzylalkohol oder 3-Phenoxy-4-fluor-benzaldehyd und Natriumcyanid, so können die beiden Verfahrensvarianten durch folgendes Formelschema skizziert werden:

(2a)

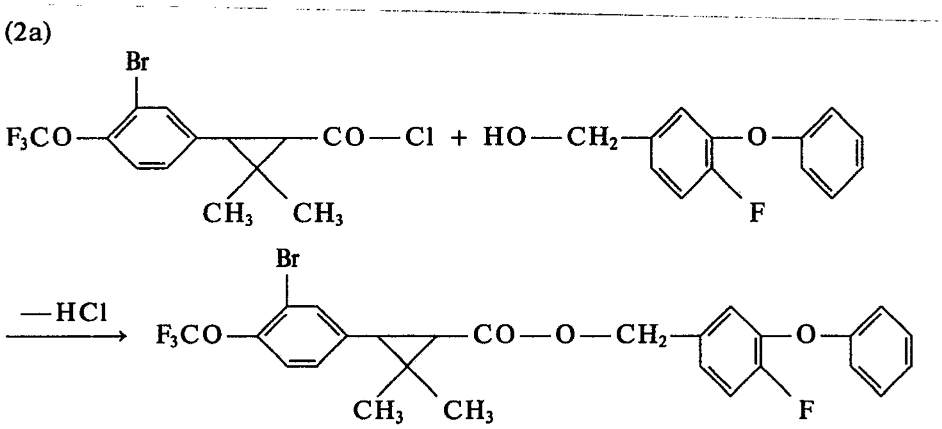


(2b)

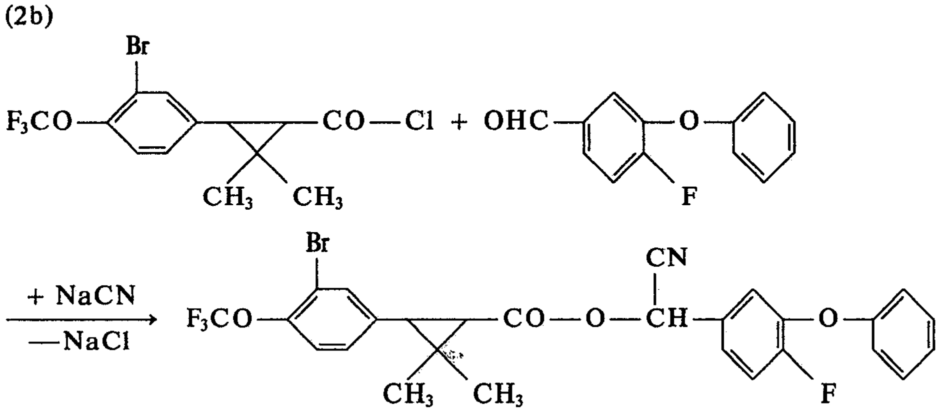


Die verschiedenen Varianten von Verfahren (2) werden im allgemeinen unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und

Dioxan, Ketone wie Aceton, Methylethyl-, Methylisopropyl- und Methylisobutylketon, Ester wie Essigsäuremethylester und -ethylester, Nitrile, wie z. B. Acetonitril und Propionitril. Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Die Verfahrensvariante (2b) wird vorzugsweise unter Verwendung von Wasser als zweiter Solvenskomponente neben einem mit Wasser nicht mischbaren organischen Lösungsmittel aus der Reihe der Kohlenwasserstoffe und gegebenenfalls in Gegenwart eines Katalysators durchgeführt. Als Katalysatoren werden gegebenenfalls Verbindungen verwendet, welche zum Transfer von Anionen aus Wasser in organische Lösungsmittel geeignet sind. Beispiele hierfür sind Benzyltriethyl-ammonium-hydrogensulfat, Tetrabutylammonium-bromid und Methyl-tricapryl-ammoniumchlorid (Aliquat 336).

Bei der Verfahrensvariante (2a) können die üblichen Säurebindemittel verwendet werden. Als Beispiele seien genannt:
Alkalihydroxide, wie z. B. Natrium- und Kaliumhydroxid, Alkalicarbonate, wie z. B. Natrium- und Kaliumcarbonat, Alkalialkohole, wie z. B. Natrium- und Kaliummethylat und -ethylat, ferner aliphatische und aromatische, auch heterocyclische Amine, wie z. B. Triethyl- und Trimethylamin, N,N-Dimethylanilin, N,N-Dimethyl-benzylamin, Pyridin, Diazabicyclooctan, Diazabicyclononan und Diazabicycloundecen.

Die Reaktionstemperatur wird bei Verfahren (2) zwischen 0 und 100°C, bei den Varianten (a) und (b) vorzugsweise zwischen 10 und 50°C gehalten. Das Verfahren wird im allgemeinen bei Normaldruck durchgeführt.

Zur Durchführung von Verfahren (2) — Varianten (a) und (b) — werden angenähert äquimolare Mengen der Ausgangsstoffe, gegebenenfalls zusammen mit Säureakzeptoren bzw. Katalysatoren, in geeigneten Verdünnungsmitteln zusammengegeben und die Reaktionsgemische werden mehrere Stunden gerührt. Zur Aufarbeitung, welche nach üblichen Methoden erfolgen kann, wird gegebenenfalls mit Wasser und/oder einem mit Wasser nicht mischbaren organischen Lösungsmittel verdünnt, die organische Phase abgetrennt, mit Wasser gewaschen, getrocknet und filtriert. Das Lösungsmittel wird unter vermindertem Druck sorgfältig abdestilliert, wobei das Rohprodukt als Rückstand verbleibt.

Die als Ausgangsstoffe zu verwendenden neuen 2,2-Dimethyl-3-phenylcylopropan-carbonsäuren sind durch die Formel (II), die entsprechenden Säurechloride durch die Formel (IIa) definiert. Vorzugsweise haben in diesen Formeln $R^1$ und $R^2$ die gleichen Bedeutungen, wie sie bei der Definition der entsprechenden Gruppen in Formel (I) als bevorzugt angegeben sind.

Die Carbonsäurechloride der Formel (IIa) erhält man aus den entsprechenden Carbonsäuren der Formel (II) nach üblichen Methoden, beispielsweise durch Umsetzung mit Thionylchlorid, gegebenenfalls unter Verwendung eines Verdünnungsmittels, wie z. B. Tetrachlorkohlenstoff bei Temperaturen zwischen 0 und 100°C und anschließende Destillation.

Als Beispiele für die Verbindungen der Formel II bzw. (IIa) seien genannt:

3-(3-Brom-4-trifluormethoxy-phenyl)-, 3-(4-Trifluormethoxy-phenyl)-, 3-(4-Trifluormethylthio-phenyl)-, 3-(3-Chlor-4-trifluormethoxy-phenyl)-, 3-(3,4-Trifluorethylendioxy-phenyl)-, 3-(3,4-Difluormethylendioxy-phenyl)-, 3-(3-Chlor-4-(2-chlor-1,1,2-trifluorethoxy)-phenyl)-, 3-(3-Chlor-4-(1,1,2,2-tetrafluorethoxy)-phenyl)-, 3-(3-Chlor-4-chlordifluormethoxy-phenyl)-, 3-(3-Chlor-4-difluormethoxy-phenyl)-2,2-dimethylcyclopropancarbonsäure und die entsprechenden Säurechloride.

Die neuen substituierten 2,2-Dimethyl-3-phenyl-cyclopropancarbonsäuren der Formel (II) erhält man nach dem unter (4) dargelegten Verfahren durch Umsetzung von substituierten 2,2-Dimethyl-3-phenyl-cyclopropancarbonsäureestern der Formel IV (oben) mit Alkalihydroxiden, wie z. B. Natrium- oder Kaliumhydroxid, in Gegenwart von Verdünnungsmitteln wie z. B. Wasser und/oder Alkoholen wie z. B. Ethanol bei Temperaturen zwischen 50 und 100°C.

Die Aufarbeitung kann nach üblichen Methoden durchgeführt werden, beispielsweise, indem man nach Verdünnen mit Wasser mit einem mit Wasser nicht mischbaren organischen Lösungsmittel extrahiert, die organische Phase trocknet, filtriert und das Lösungsmittel unter vermindertem Druck sorgfältig abdestilliert, wobei man das Produkt als Rückstand erhält.

Die Verbindungen der Formel (III) sind bekannt (vergleiche US-PS 4 163 787, US-PS 3 835 176 und DE-OS 2 709 264).

Bevorzugte Ausgangsstoffe der Formel (III) sind beispielsweise 4-Fluor-3-phenoxy-benzylalkohol, 4-Fluor-3-phenoxy-benzaldehyd, $\alpha$-Cyano-4-fluor-3-phenoxy-benzylalkohol.

Die als Zwischenprodukte einzusetzenden neuen substituierten 2,2-Dimethyl-3-phenyl-cyclopropancarbonsäureester sind durch die Formel (IV) definiert.

Vorzugsweise haben darin $R^1$ und $R^2$ die gleichen Bedeutungen, wie sie bei der Definition der entsprechenden Gruppen in Formel (I) als bevorzugt angegeben sind. $R^6$ steht in dieser Formel bevorzugt für Methyl oder Ethyl.

Als Beispiele für die Verbindungen der Formel (IV) seien genannt:

3-(3-Brom-4-trifluormethoxy-phenyl)-, 3-(4-Trifluormethoxy-phenyl)-, 3-(4-Trifluormethylthio-phenyl)-, 3-(3-Chlor-4-trifluormethoxy-phenyl)-, 3-(3,4-Trifluorethylendioxy-phenyl)-, 3-(3,4-Difluormethylendioxy-phenyl)-, 3-(3-Chlor-4-(2-chlor-1,1,2-trifluorethoxy)-phenyl)-, 3-(3-Chlor-4-(1,1,2,2-tetrafluorethoxy)-phenyl), 3-(3-Chlor-4-chlordifluormethoxy-phenyl)-, 3-(3-Chlor-4-difluormethoxy-phenyl)-2,2-dimethyl-cyclopropancarbonsäure-methylester und die entsprechenden Ethylester.

Man erhält die neuen substituierten 2,2-Dimethyl-3-phenyl-cyclopropancarbonsäureester der Formel (IV) nach dem unter (6) dargelegten Verfahren, indem man substituierte 2-Methyl-3-phenyl-propene der Formel (V) mit Diazoessigsäureestern der Formel (VI), in Gegenwart von Katalysatoren umsetzt.

Als solche kommen Kupfer und Kupferverbindungen verschiedener Oxidationsstufen — auch Gemische — in Frage, wie z. B. Kupfer (als Pulver oder Bronze), Kupfer(I)- und -(II)-chlorid, Kupfer(I)- und -(II)-oxid sowie Kupfer(II)sulfat. Die Reaktionstemperatur kann innerhalb eines größeren Bereichs variiert werden. Im allgemeinen arbeitet man zwischen 50 und 200° C, vorzugsweise bei 80 bis 150° C.

Nach Ende der Stickstoffentwicklung wird das Reaktionsgemisch abgekühlt, mit einem mit Wasser nicht mischbaren Lösungsmittel, wie z. B. Methylenchlorid, verdünnt und filtriert. Das Filtrat wird mit Wasser gewaschen, getrocknet und destillativ aufgearbeitet.

Die als Zwischenprodukte zu verwendenden neuen substituierten 2-Methyl-3-phenyl-propene sind durch die Formel (V) definiert.

Vorzugsweise haben darin $R^1$ und $R^2$ die gleichen Bedeutungen, wie sie bei der Definition der entsprechenden Gruppen in Formel (I) als bevorzugt angegeben sind.

Als Beispiele seien genannt:

3-(3-Brom-4-trifluormethoxy-phenyl)-,
3-(4-Trifluormethoxy-phenyl)-,
3-(4-Trifluormethylthio-phenyl)-,
3-(3-Chlor-4-trifluormethoxy-phenyl)-,
3-(3,4-Trifluorethylendioxy-phenyl)-,
3-(3,4-Difluormethylendioxy-phenyl)-,
3-(3-Chlor-4-(2-chlor-1,1,2-trifluorethoxy)-phenyl)-,
3-(3-Chlor-4-(1,1,2,2-tetrafluorethoxy)-phenyl)-,
3-(3-Chlor-4-chlordifluormethoxy-phenyl)-,
3-(3-Chlor-4-difluormethoxy-phenyl)-2-methyl-1-propen.

Die Diazoessigsäureester der Formel (VI) sind literaturbekannte Verbindungen oder können analog bekannter Verfahren hergestellt werden. Als Beispiele seien genannt: Diazoessigsäure-methylester und -ethylester.

Die neuen substituierten 2-Methyl-3-phenyl-propene der Formel (V) erhält man nach dem unter (8) dargelegten Verfahren. Dieses Verfahren wird nach den bei Carbonyl-Olefinierungen nach Wittig üblichen Methoden durchgeführt.

Die Herstellung des Phosphorans (VIII) ist bekannt.

Die als Zwischenprodukte zu verwendenden substituierten Benzaldehyde sind teilweise bekannt (vergleiche z. B. J.Gen.Chem. USSR 30 (1960), 3103; Bull.Soc.Chim. France 1955, 1594 ibid. 1962, 254—262; J.Org.Chem. 37 (1972), 673; DE-OS 2 029 556; US-PS 3 387 037; J.Med.Chem. 16 (1973), 1399) und sind durch die Formel (VII) definiert.

Benzaldehyde, in denen $R^1$ die gleiche Bedeutung hat, wie sie bei der Definition der entsprechenden Gruppe in Formel (I) als bevorzugt angegeben sind und $R^2$ für Brom steht, sind neu.

Als Beispiele seien genannt:

3-Brom-4-trifluormethoxy-,
4-Trifluormethoxy-,
4-Trifluormethylthio-,
3-Chlor-4-trifluormethoxy-,
3,4-Trifluormethylendioxy-,
3,4-Difluormethylendioxy-,
3-Chlor-4-(1,1,2,2-tetrafluorethoxy)-,
3-Chlor-4-(2-chlor-1,1,2-trifluorethoxy)-,
3-Chlor-4-chlordifluormethoxy-,
3-Chlor-4-difluormethoxy-benzaldehyd.

Man erhält die neuen substituierten Benzaldehyde der Formel (VII) nach dem unter (10) dargelegten Verfahren. Das Verfahren zur Herstellung von Verbindungen der Formel VII (oben) wird vorzugsweise unter Verwendung von Verdünnungsmitteln durchgeführt. Als solche kommen gegebenenfalls Wasser und/oder aliphatische oder aromatische, gegebenenfalls halogenierte Kohlenwasserstoffe, wie z. B. Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol in Frage.

Die Reaktionstemperatur liegt zwischen 0 und 100° C, vorzugsweise zwischen 10 und 60° C. Das Verfahren wird gewöhnlich bei Normaldruck durchgeführt.

In einer bevorzugten Variante (a) von Verfahren (10) wird als Oxidationsmittel Chrom(VI)oxid-Pyridin-Chlorwasserstoff (1/1/1) verwendet. Zur Aufarbeitung wird, gegebenenfalls nach Abdekantieren von ungelösten Komponenten, destilliert.

In einer zweiten bevorzugten Variante (b) von Verfahren (10) wird Salpetersäure als Oxidationsmittel und Wasser als Verdünnungsmittel verwendet. Zur Aufarbeitung wird mit Natronlauge alkalisiert, mit einem mit Wasser nicht mischbaren Lösungsmittel, wie z. B. Toluol, extrahiert, oder Extrakt mit Wasser gewaschen, getrocknet, filtriert und destilliert.

In einer dritten bevorzugten Variante (c) von Verfahren (10) wird Chromsäure bzw. Dichromat und Schwefelsäure als Oxidationsmittel verwendet. Verdünnungsmittel ist hierbei vorzugsweise ein Zweiphasensystem aus Wasser und einem der oben genannten organischen Lösungsmittel. Als Katalysatoren werden vorzugsweise Verbindungen verwendet, welche zum Transfer von Anionen aus Wasser in organische Lösungsmittel geeignet sind. Beispiele hierfür sind Benzyl-triethyl-ammonium-hydrogensulfat, Tetrabutyl-ammonium-bromid und Methyl-tricapryl-ammoniumchlorid (Aliquat 336).

Zur Aufarbeitung wird mit Wasser verdünnt, die organische Phase abgetrennt, mit Wasser gewaschen, getrocknet, filtriert und destilliert.

Die als Zwischenprodukte einzusetzenden substituierten Benzole sind durch die Formel (IX) definiert. Vorzugsweise haben darin $R^1$ und $R^2$ die gleichen Bedeutungen, wie sie bei der Definition der entsprechenden Gruppen in Formel (I) als bevorzugt angegeben sind und $R^7$ steht für Hydroxymethyl.

Als Beispiele für Verbindungen der Formel IX seien genannt:

3-Brom-4-trifluormethoxy-,
4-Trifluormethoxy-,
4-Trifluormethylthio-,
3-Chlor-4-trifluormethoxy-,
3,4-Trifluorethylendioxy-,
3,4-Difluormethylendioxy-,
3-Chlor-4-(2-chlor-1,1,2-trifluorethoxy)-,
3-Chlor-4-(1,1,2,2-tetrafluorethoxy)-,
3-Chlor-4-chlor-difluormethoxy-,
3-Chlor-4-difluormethoxy-1-hydroxy-methyl-benzol.

Die Benzole der Formel (IX) sind teilweise bekannt bzw. können sie auf bekannte Art und Weise hergestellt werden (vergleiche DE-OS 2 333 849).

Man erhält sie z. B., indem man die entsprechenden Carbonsäurehalogenide z. B. die Fluoride in Gegenwart von Natriumboranat, in Gegenwart von Verdünnungsmitteln wie z. B. Dioxan, bei Temperaturen zwischen 20 und 150° C, reduziert (vergleiche auch die Herstellungsbeispiele).

Die Einführung der Halogenatome z. B. der Bromatome in den Benzolkern der für die oben erläuterte Reduktion verwendeten Carbonsäurehalogenide geschieht nach bekannten Methoden (vergleiche Houben-Weyl, Methoden der organischen Chemie, Band 5/3, S. 215) z. B. durch Diazotieren entsprechender Aminobenzoesäuren in Bromwasserstoffsäure-Lösung und anschließendes Verkochen. Die Carbonsäuren werden anschließend in die entsprechende Carbonsäurehalogenide überführt (vergleiche auch die Herstellungsbeispiele).

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopada z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrate, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni,

Saissetia oleae, Laodelphax striatellus, Nilaparvata lugens, Aonidiella aurantii, Aspidiotus hederae, Pseudococcus spp. Psylla spp.
Aus der Ordnung der Lepidoptera z. B. Pectionophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotassa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und syntheti-

sche pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

## Herstellungsbeispiele

### Beispiel 1

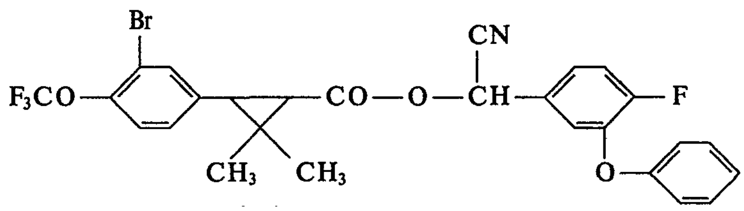

2,62 g (0,012 Mol) 4-Fluor-3-phenoxy-benzaldehyd und 4,5 g (0,012 Mol) (+)cis/trans 3-(3-Brom-4-trifluormethoxyphenyl)-2,2-dimethyl-cyclopropancarbonsäurechlorid werden zusammen unter Rühren bei 20—25°C zu einer Mischung von 1,0 g Natriumcyanid, 1,5 ml Wasser, 50 ml Gyclohexan und 0,3 g Tetrabutylammoniumbromid getropft und 4 Stunden bei 20—25°C gerührt. Anschließend wird die Reaktionsmischung mit 300 ml Toluol versetzt und zweimal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 mm Hg Badtemperatur entfernt. Man erhält 4,0 g (57,7% der Theorie) (+)cis/trans 3-(3-Brom-4-trifluormethoxy-phenyl)-2,2-dimethyl-cyclopropancarbonsäure-(4-fluor-3-phenoxy-α-cyano-benzyl)-ester als zähes Öl.

Die Struktur wird durch das $^{1}H$-NMR-Spektrum bestätigt.
$^{1}H$-NMR in $COCL_3$/TMS, τ (ppm);

Aromatische H: 2,5—3,25 (m/11 H)

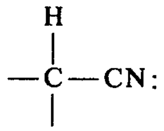 3,6–3,8 (m/l H)

Cyclopropan-H: 7,2—8,2 (m/2 H)
Dimethyl-H: 8,55—9,15 (m/6 H)

## Beispiel 2

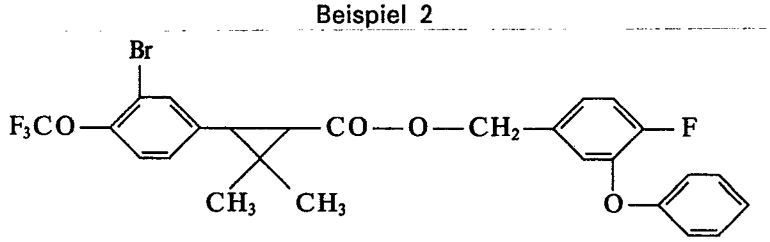

2,64 g (0,012 Mol) 4-Fluor-3-phenoxy-benzylalkohol und 4,5 g (0,012 Mol) (+)cis/trans 3-(3-Brom-4-trifluormethoxy-phenyl)-2,2-dimethyl)-cyclopropancarbonsäurechlorid werden in 100 ml wasserfreiem Toluol gelöst. Bei einer Temperatur von 20—25° C wird 1,2 g Pyridin gelöst in 10 ml wasserfreiem Toluol, unter Rühren zugetropft. Anschließend wird 4 weitere Stunden bei 25—35° C gerührt. Das Reaktionsgemisch wird in 150 ml Wasser, dem 10 ml konzentrierter Salzsäure zugesetzt werden, gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60° C/1 mm Hg Badtemperatur entfernt. Man erhält 5,7 g (85,9% der Theorie) (+)cis/trans 3-(3-Brom-4-trifluormethoxyphenyl)-2,2-dimethyl-cyclopropancarbonsäure-(4-fluor-3-phenoxy-benzyl)-ester als gelbes Öl.

Die Struktur wird durch das $^{1}H$-NMR-Spektrum bewiesen.
$^{1}H$-NMR- in $CDCl_3$/TMS, τ (ppm):

Aromatische-H: 2,5—3,2 (m/11 H)
Benzyl-H: 4,95 (S) und 5,05 (S) insgesamt 2 H
Cyclopropan-H: 7,3—8,20 (m/2 H)
Dimethyl-H: 8,6—9,15 (m/6 H)

Analog Beispiel 1 und 2 lassen sich folgende Verbindungen der Formel (I) herstellen:

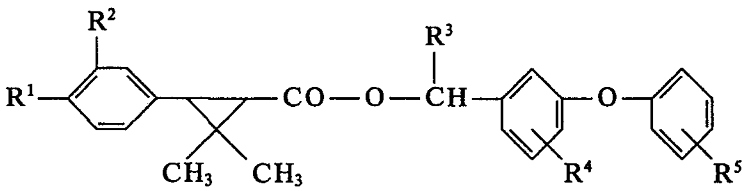

| Beispiel Nr. | $R^1$ | $R^2$ | $R^3$ | $R^4$ | $R^5$ | $^1$H-NMR in $CDCl_3$/TMS, $\tau$ (ppm) |
|---|---|---|---|---|---|---|
| 3 | $F_3CO$ | H | CN | 4-F | H | 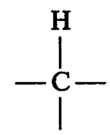 CN: 3,57-3,80 (m/1 H) |
| 4 | $F_3CO$ | H | H | 4-F | H | Benzyl-H: 4,88 (S) 5,05 (S)/2 H |
| 5 | $F_3CS$ | H | CN | 4-F | H | |
| | $F_3CS$ | H | H | 4-F | H | |

Die Strukturen werden durch die $^1$H-NMR-Spektren bestätigt.

## Ausgangsprodukte

### Beispiel a

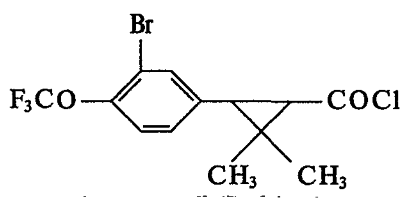

9,6 g (0,027 Mol) 3-(3-Brom-4-trifluormethoxyphenyl)-2,2-dimethyl-cyclopropancarbonsäure werden in 100 ml Tetrachlorkohlenstoff gelöst und bei 60°C langsam unter Rühren 10 g Thionylchlorid zugetropft. Anschließend wird 4 Stunden zum Rückfluß erhitzt. Nach beendeter Reaktionszeit werden überschüssiges Thionylchlorid sowie Tetrachlorkohlenstoff im Wasserstrahlvakuum abdestilliert. Man erhält 9,4 g (93,7% der Theorie) 3-(3-Brom-4-trifluormethoxyphenyl)-2,2-dimethyl-cyclopropancarbonsäurechlorid als gelbe Flüssigkeit.

Analog Beispiel (a) werden die übrigen Säurechloride der Formel II(a) erhalten.

### Beispiel b

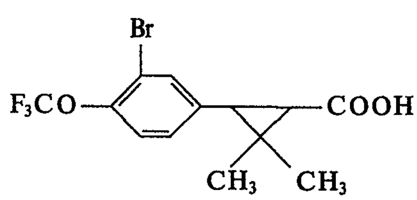

16 g (0,042 Mol) 3-(3-Brom-4-trifluormethoxyphenyl)-2,2-dimethyl-cyclopropancarbonsäureethylester werden in 50 ml Ethanol gelöst, dann mit einer Lösung von 3,4 g (0,085 Mol) Natriumhydroxid in 50 ml Wasser versetzt und 4 Stunden unter Rühren zum Rückfluß erhitzt. Anschließend wird das Ethanol im Wasserstrahlvakuum abdestilliert, der Rückstand in 300 ml Wasser aufgenommen und 1mal mit 300 ml Methylenchlorid extrahiert. Die wäßrige Phase wird abgetrennt, mit konzentrierter Salzsäure angesäuert und anschließend mit 2mal 300 ml Methylenchlorid extrahiert. Die organische Phase wird dann abgetrennt, über Magnesiumsulfat getrocknet und das Lösungsmittel im Wasserstrahlvakuum abdestilliert. Letzte Lösungsmittelreste werden durch kurzes Andestillieren bei 2 mm Hg/50°C

Badtemperatur entfernt. Man erhält dann 9,8 g (66,3% der Theorie) (+)cis/trans-3-(3-Brom-4-trifluormethoxyphenyl)-2,2-dimethyl-cyclopropancarbonsäure als gelbes zähes Öl. Die Struktur wird durch das $^1$H-NMR-Spektrum bestätigt.

Analog Beispiel (b) erhält man die folgenden Verbindungen der Formel (II):

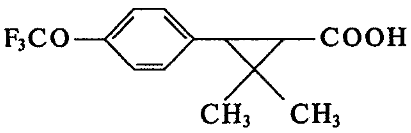

Ausbeute: 65% der Theorie

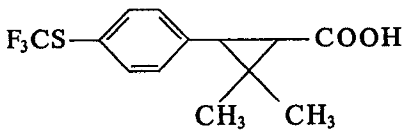

Beispiel c

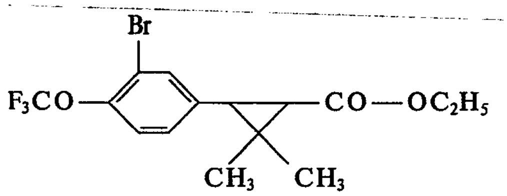

Eine Mischung von 33,3 g (0,11 Mol) 1-(3-Brom-4-trifluormethoxyphenyl)-2-methyl-1-propen, 1,2 g Kupferpulver und 0,9 g Kupfersulfat (wasserfrei) wird auf 120—130°C erhitzt und dann unter Rühren ebenfalls bei 120—130°C eine Mischung von 16,6 g (0,056 Mol) 1-(3-Brom-4-trifluormethoxyphenyl)-2-methyl-1-propen und 19,4 g (0,17 Mol) Diazoessigsäureethylester, sehr langsam, innerhalb von 6 Stunden, zugetropft. Nach beendeter Stickstoffentwicklung wird die Mischung abgekühlt, mit 500 ml Methylenchlorid verdünnt und dann filtriert.

Das Filtrat wird mit 500 ml Wasser ausgeschüttelt, anschließend wird die organische Phase abgetrennt, über Magnesiumsulfat getrocknet und dann das Lösungsmittel im Wasserstrahlvakuum abdestilliert. Der ölige Rückstand wird im Vakuum destilliert. Man erhält dabei zwei Fraktionen:

Fraktion 1: Siedepunkt 100—110°C/10 mm Hg
Fraktion 2: Siedepunkt 120—160°C/10 mm Hg

Fraktion 1 erweist sich als nicht umgesetztes 1-(3-Brom-4-trifluormethoxyphenyl)-2-methyl-1-propen.

Fraktion 2 wird nochmals destilliert. Man erhält 8 g (13% der Theorie) (+)cis/trans 3-(3-Brom-4-trifluormethoxyphenyl)-2,2-dimethyl-cyclopropancarbonsäureethylester als farblose Flüssigkeit mit dem Siedepunkt 145—160°C/10 mm Hg.

Analog Beispiel (c) erhält man die folgenden Verbindungen der Formel (IV):

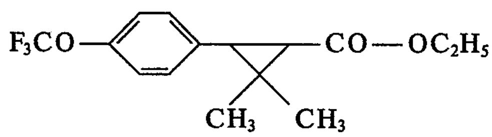

Ausbeute: 19% der Theorie
Kp.: 115—125° C/3 mm Hg

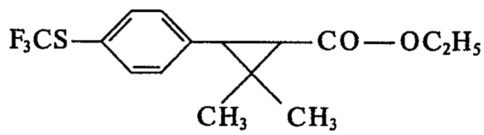

Ausbeute: 20% der Theorie
Kp.: 125—130° C/5 mm Hg

Beispiel d

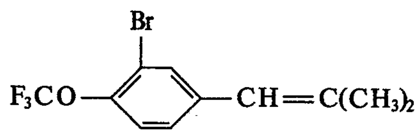

Zu einer Suspension von 129,6 g (0,3 Mol) trockenem Isopropyl-triphenylphosphoniumjodid in 500 ml wasserfreiem Tetrahydrofuran werden unter Stickstoff bei 0° C und unter Rühren 150 ml einer 20%igen Lösung von n-Butyllithium in n-Hexan zugetropft. Die so erhaltene tiefrote Lösung wird noch 15 Minuten bei 0° C gerührt und dann bei 0—10° C 80,7 g (0,3 Mol) 3-Brom-trifluormethoxybenzaldehyd zugetropft. Anschließend wird solange bei Raumtemperatur gerührt, bis Entfärbung eingetreten ist (ca. 12 Stunden). Das Reaktionsgemisch wird dann mit 900 ml Wasser versetzt und 5mal mit je 200 ml Petrolether extrahiert. Die Petroletherphasen werden über Magnesiumsulfat getrocknet und dann das Lösungsmittel am Rotationsverdampfer im Wasserstrahlvakuum abgezogen. Der Rückstand wird mit 150 ml n-Hexan versetzt, gut verrührt und dann filtriert. Anschließend wird vom Filtrat bei Normaldruck das Lösungsmittel abdestilliert und der ölige Rückstand dann im Vakuum destilliert. Man erhält 50 g (56,5% der Theorie) 1-(3-Brom-4-trifluormethoxyphenyl)-2-methyl-1-propen als leicht gelbliche Flüssigkeit mit dem Siedepunkt 107—108° C 10 mm Hg.

Analog Beispiel (d) erhält man folgende Verbindungen der Formel (V).:

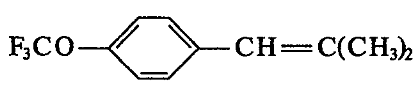

Ausbeute: 44% der Theorie
Kp.: 65—67° C/4 mm Hg

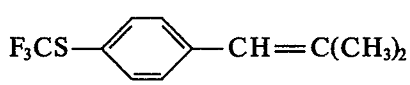

Ausbeute: 53% der Theorie
Kp.: 95—96° C/10 mm Hg

Beispiel e

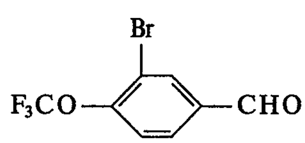

Zu einer Lösung von 27 mg (0,1 Mol) 3-Brom-4-trifluormethoxybenzylalkohol in 250 ml Methylenchlorid gibt man bei Raumtemperatur eine Mischung aus 29,4 g (0,3 Mol) Schwefelsäure, 50 ml Wasser und 2 ml Aliquat 336 (Tricaprylmethylammoniumchlorid). Danach versetzt man Reaktionsgemisch mit 9,7 g (0,033 Mol) Kaliumdichromat und hält die Temperatur während 2 Stunden durch schwaches Kühlen auf ca. 25° C. Nach Zugabe von 100 ml Wasser trennt man die organische Phase ab und schüttelt das

Wasser noch einmal mit 100 ml Methylenchlorid aus. Die organischen Phasen werden 2mal mit je 100 ml Wasser, dann einmal mit 100 ml gesättigter Natriumhydrogencarbonatlösung und noch einmal mit 100 ml Wasser gewaschen, getrocknet über Natriumsulfat und im Vakuum eingedampft. Der Rückstand wird destilliert. Man erhält auf diese Weise 20,4 g (75,8% der Theorie) 3-Brom-4-trifluormethoxybenzaldehyd als farbloses Öl mit dem Siedepunkt 97° C/10 mm Hg.

Beispiel f

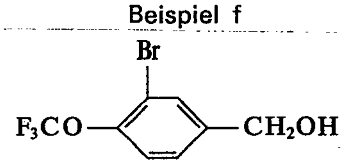

Man legt 764 ml Dioxan und 53 g Natriumboranat vor und tropft bei Rückflußtemperatur 400 g 3-Brom-4-trifluormethoxybenzoylfluorid in 364 ml Dioxan innerhalb von 11 Stunden zu. Es wird 1 Stunde bei Rückflußtemperatur nachgerührt. Man kühlt ab und gibt den Ansatz auf Eiswasser. Man stellt mit Salzsäure sauer und nimmt den organischen Anteil in Methylenchlorid auf. Die wäßrige Schicht wird zweimal mit Methylenchlorid extrahiert und die vereinigten organischen Phasen über Natriumsulfat getrocknet. Anschließend wird destilliert. Das gewünschte Produkt 3-Brom-4-trifluormethoxy-benzylalkohol hat einen Siedepunkt von 132° C/20 mm Hg und einen Schmelzpunkt von 57—58° C.

Beispiel g

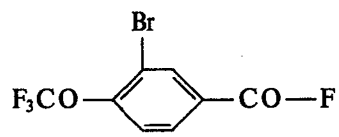

100 ml wasserfreie Fluorwasserstoffsäure wird auf —10° C abgekühlt. Bei —5 bis 0° C tropft man unter zügiger Chlorwasserstoff-Entwicklung 420 g 3-Brom-4-trifluormethoxy-benzoylchlorid in zwei Stunden zu. Nach Abklingen der Chlorwasserstoff-Entwicklung läßt man die Temperatur ansteigen und bei 20° C ausreagieren. Die überschüssige Fluorwasserstoffsäure wird abdestilliert. Der Rückstand wird durch Destillation gereinigt. Man erhält 352,8 g (88,7% der Theorie) 3-Brom-4-trifluormethoxybenzoylfluorid mit einem Brechungsindex von $n_D^{20}$ : 1,4760.

Beispiel h

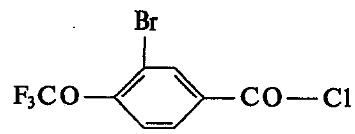

Man legt 462 g 3-Brom-4-trifluormethoxybenzoesäure und 358 ml Thionylchlorid gemeinsam vor und heizt entsprechend der Gasentwicklung bis auf 70° C auf. Man rührt, bis die Gasentwicklung beendet ist und destilliert das überschüssige Thionylchlorid bei Normaldruck ab. Der Rückstand wird destilliert. Das 3-Brom-4-trifluor-methoxybenzoesäurechlorid siedet bei 111° C/20 mm Hg und hat einen Brechungsindex von $n_D^{20}$ : 1,5120.

Beispiel i

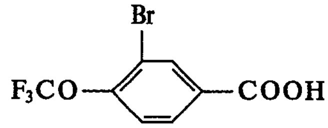

44 g 3-Amino-4-trifluormethoxybenzoesäure werden in eine Mischung aus 440 ml Wasser und 220 ml 48%ige Bromwasserstoffsäure-Lösung eingerührt und bei 0° bis 3° C mit 14 g Natriumnitrat in 40 ml Wasser diazotiert. Es wird 15 Minuten nachgerührt und der Nitritüberschuß mit Amidosulfonsäure vernichtet. Anschließend wird mit 300 ml Eiswasser verdünnt. Diese Diazolösung läßt man bei 20° C in eine frisch zubereitete Lösung aus 10 g Kupfer(I)bromid in 200 ml 48%iger Bromwasserstoffsäure-Lösung laufen. Die entstandenen Kristalle werden abgesaugt und getrocknet. Nach dem Umkristallisieren aus Chlorbenzol erhält man die 3-Brom-4-trifluormethoxybenzoesäure mit einem Schmelzpunkt von 124—125° C.

## Beispiel A

### Phaedon-Larven-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewüschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: 1, 3 und 4.

## Beispiel B

### Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: (3)

## Beispiel C

### Grenzkonzentrations-Test/Bodeninsekten

Testinsekt: Agrotis segetum Larven (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vemischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 3

Beispiel D

Invitro-Tauchtest/Ektoparasiten

Test mit Boophilus microplus resistent

Lösungsmittel:
35 Gewichtsteile Ethylenglykolmonomethylether
33 Gewichtsteile Nonylphenolpolyglycolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik: 4, 3, 1 und 2.

## Patentansprüche

1. Substituierte Phenyl-cyclopropancarbonsäureester der Formel

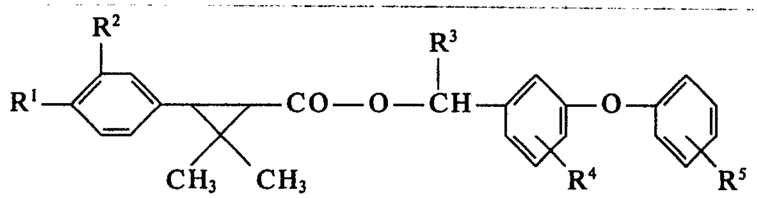

(I)

in welcher

$R^1$ für einfach oder mehrfach, gleich oder verschieden halogensubstituierte Alkoxy- oder Alkylthioreste steht,
$R^2$ für Wasserstoff oder Halogen steht,
$R^1$ und $R^2$ gemeinsam für einfach oder mehrfach, gleich oder verschieden halogen-substituiertes Alkylendioxy stehen,
$R^3$ für Wasserstoff, oder Cyano steht,
$R^4$ für Halogen steht, und
$R^5$ für Wasserstoff oder Halogen steht.

2. Verfahren zur Herstellung der neuen Verbindungen der Formel I

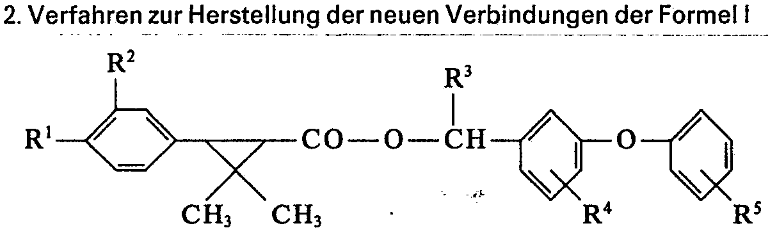

(I)

in welcher

$R^1$ für einfach oder mehrfach, gleich oder verschieden halogensubstituierte Alkoxy- oder Alkylthioreste steht,
$R^2$ für Wasserstoff oder Halogen steht,
$R^1$ und $R^2$ gemeinsam für einfach oder mehrfach, gleich oder verschieden halogen-substituiertes Alkylendioxy stehen,
$R^3$ für Wasserstoff, oder Cyano steht,
$R^4$ für Halogen steht, und
$R^5$ für Wasserstoff oder Halogen steht,

dadurch gekennzeichnet, daß man substituierte 2,2-Dimethyl-3-phenyl-cyclopropancarbonsäuren der Formel II

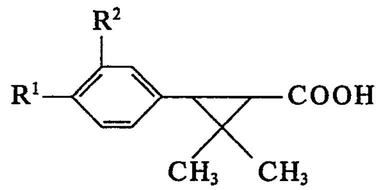

(II)

in welcher

$R^1$ und $R^2$ die oben angegebenen Bedeutungen haben,

— oder reaktionsfähige Derivate derselben, mit 3-Phenoxy-benzyl-alkoholen der Formel III

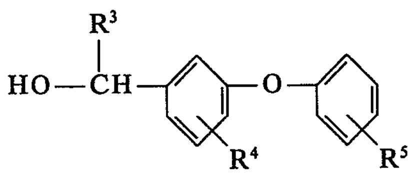

in welcher

$R^3$, $R^4$, $R^5$ die oben angegebenen Bedeutungen haben,

oder mit deren reaktionsfähigen Derivaten, gegebenenfalls in Gegenwart eines Säureakzeptors und/oder eines Katalysators und gegebenenfalls unter Verwendung eines oder mehrerer Verdünnungsmittel umsetzt.

3. Substituierte 2,2-Dimethyl-3-phenyl-cyclopropancarbonsäuren der Formel II

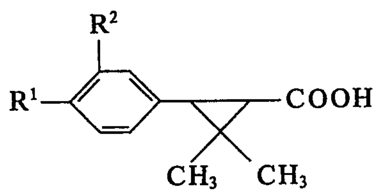

(II)

in welcher

$R^1$ für einfach oder mehrfach, gleich oder verschieden halogen-substituierte $C_1-C_4$-Alkoxy- oder $C_1-C_4$-Alkylthioreste steht,

$R^2$ für Wasserstoff oder Halogen steht oder

$R^1$ und $R^2$ gemeinsam für einfach oder mehrfach, gleich oder verschieden halogen-substituiertes Alkylendioxy stehen.

4. Verfahren zur Herstellung der neuen Verbindungen der Formel (II) gemäß Anspruch 3, dadurch gekennzeichnet, daß man substituierte 2,2-Dimethyl-3-phenyl-cyclopropancarbonsäureester der Formel (IV)

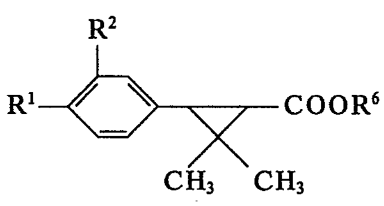

(IV)

in welcher

$R^1$ und $R^2$ die oben angegebenen Bedeutungen haben und

$R^6$ für $C_1-C_4$-Alkyl steht,

mit Alkalihydroxiden in Gegenwart von Verdünnungsmitteln auf Temperaturen zwischen 50 und 200°C erhitzt und anschließend bei Raumtemperatur mit Mineralsäuren ansäuert.

5. Substituierte Phenylcyclopropancarbonsäureester der Formel (I) gemäß Anspruch 1, dadurch gekennzeichnet, daß

$R^1$ für $C_1-C_2$-Fluoralkoxy, $C_1-C_2$-Chlorfluoralkoxy, $C_1-C_2$-Fluoralkylthio oder $C_1-C_2$-Chlorfluoralkylthio steht,
$R^2$ für Wasserstoff, Chlor oder Brom steht,
$R^1$ und $R^2$ gemeinsam für $C_1-C_2$-Fluoralkylendioxy stehen,
$R^3$ für Wasserstoff, Cyano steht
$R^4$ für Fluor, Chlor oder Brom steht, und
$R^5$ für Wasserstoff, Fluor, Chlor oder Brom steht.

6. Substituierten Phenyl-cyclopropancarbonsäureester der Formel (I), gemäß Anspruch 1, dadurch gekennzeichnet, daß

$R^1$ für Chlortrifluorethoxy, Tetrafluorethoxy, Chlordifluormethoxy, Trifluormethoxy, Trifluormethylthio und Difluormethoxy steht,
$R^2$ für Wasserstoff, Chlor oder Brom steht,
$R^1$ und $R^2$ gemeinsam für Trifluorethylendioxy oder Difluormethylendioxy stehen,
$R^3$ für Wasserstoff oder Cyano steht,
$R^4$ für 4-Fluor, 4-Chlor oder 4-Brom steht und,
$R^5$ für Wasserstoff steht.

7. Schädlingsbekämpfungsmittel, gekennzeichnet durch einen Gehalt an mindestens einem substituierten Phenyl-cyclopropancarbonsäureester der Formel I.

8. Verwendung von substituierten Phenyl-cyclopropancarbonsäureestern der Formel (I) zur Bekämpfung von Schädlingen.

**Claims**

1. Substituted phenyl-cyclopropanecarboxylic acid esters of the formula

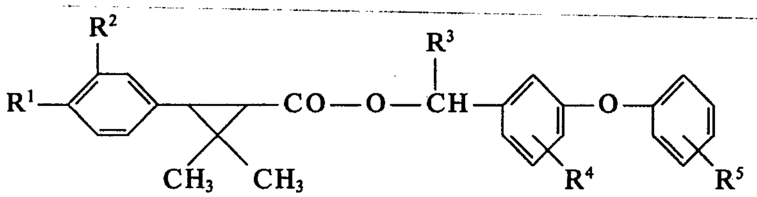

(I)

in which

$R^1$ represents alkoxy or alkylthio radicals which are mono- or polysubstituted by identical or different halogen substituents,
$R^2$ represents hydrogen or halogen,
$R^1$ and $R^2$ together represent alkylenedioxy which is mono- or polysubstituted by identical or different halogen substituents,
$R^3$ represents hydrogen or cyano,
$R^4$ represents halogen, and
$R^5$ represents hydrogen or halogen.

2. Process for the production of the new compounds of the formula I

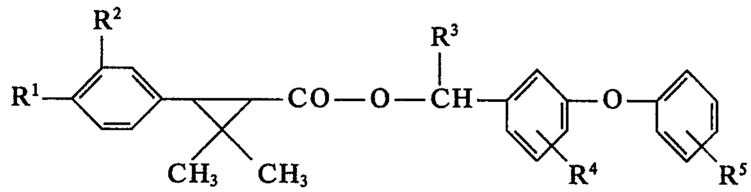

(I)

in which

$R^1$ represents alkoxy or alkylthio radicals which are mono- or polysubstituted by identical or different halogen substituents,
$R^2$ represents hydrogen or halogen,
$R^1$ and $R^2$ together represent alkylendioxy which is mono- or polysubstituted by identical or different halogen substituents,
$R^3$ represents hydrogen or cyano,
$R^4$ represents halogen and
$R^5$ represents hydrogen or halogen,

characterised in that substituted 2,2-dimethyl-3-phenyl-cyclopropane-carboxylic acids of the formula II

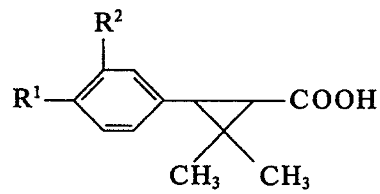

(II)

in which

$R^1$ and $R^2$ have the meanings indicated above,
— or reactive derivatives thereof, are reacted with 3-phenoxy-benzyl alcohols of the formula III

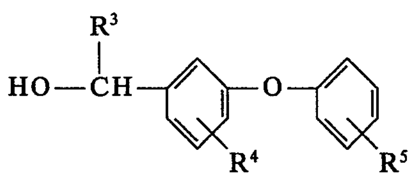

in which

$R^3$, $R^4$ and $R^5$ have the meanings given above,
or with reactive derivatives thereof, if appropriate in the presence of an acid acceptor and/or a catalyst and, if appropriate, using one or more diluents.

3. Substituted 2,2-dimethyl-3-phenyl-cyclopropanecarboxylic acids of the formula II

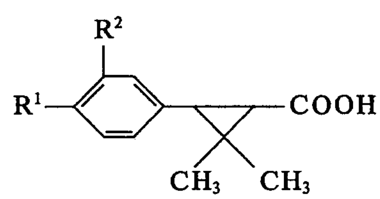

(II)

in which

$R^1$ represents $C_1-C_4$-alkoxy or $C_1-C_4$-alkylthio radicals which are mono- or polysubstituted by identical or different halogen substituents,
$R^2$ represents hydrogen or halogen or
$R^1$ and $R^2$ together represent alkylenedioxy which is mono- or polysubstituted by identical or different halogen substituents.

4. A process for the production of the new compounds of the formula (II) according to Claim 3, characterised in that substituted 2,2-dimethyl-3-phenyl-cyclopropanecarboxylic acid esters of the formula (IV)

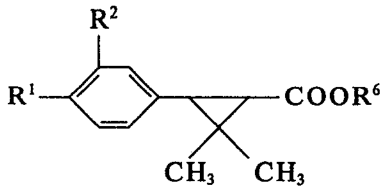

(IV)

in which

$R^1$ and $R^2$ have the meanings given above and
$R^6$ represents $C_1-C_4$-alkyl,

are heated with alkali metal hydroxides in the presence of diluents to temperatures between 50 and 200° C, and the mixtures are then acidified with mineral acids at room temperature.

5. Substituted phenylcyclopropanecarboxylic acid esters of the formula (I) according to Claim 1, characterised in that

$R^1$ represents $C_1-C_2$-fluoroalkoxy, $C_1-C_2$-chlorofluoroalkoxy, $C_1-C_2$-fluoroalkylthio or $C_1-C_2$-chlorofluoroalkylthio,
$R^2$ represents hydrogen, chlorine or bromine,
$R^1$ and $R^2$ together represent $C_1-C_2$-fluoroalkylenedioxy,
$R^3$ represents hydrogen or cyano,
$R^4$ represents fluorine, chlorine or bromine, and
$R^5$ represents hydrogen, fluorine, chlorine or bromine.

6. Substituted phenyl-cyclopropanecarboxylic acid esters of the formula (I), according to Claim 1, characterised in that

$R^1$ represents chlorotrifluoroethoxy, tetrafluoroethoxy, chlorodifluoromethoxy, trifluoromethoxy, trifluoromethylthio and difluoromethoxy,
$R^2$ represents hydrogen, chlorine or bromine,
$R^1$ and $R^2$ together represent trifluoroethylenedioxy or difluoromethylenedioxy,
$R^3$ represents hydrogen or cyano,
$R^4$ represents 4-fluoro, 4-chloro or 4-bromo, and
$R^5$ represents hydrogen.

7. Agents for combating pests, characterised in that they contain at least one substitued phenyl-cyclopropanecarboxylic acid ester of the formula I.

8. Use of substituted phenyl-cyclopropanecarboxylic acid esters of the formula (I) for combating pests.

**Revendications**

1. Esters d'acides phénylcyclopropanecarboxyliques substitués, de formule

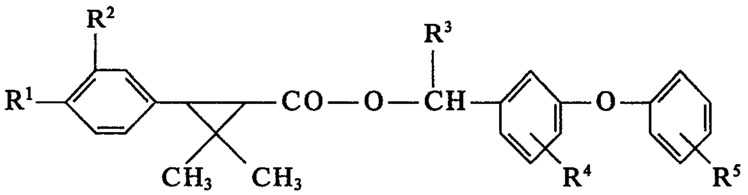

(I)

dans laquelle

$R^1$ représente des restes alkoxy ou alkylthio portant un ou plusieurs substituants halogéno égaux ou différents,
$R^2$ est l'hydrogène ou un halogène,
$R^1$ et $R^2$ forment ensemble un groupe alkylènedioxy portant un ou plusieurs substituants halogéno égaux ou différents,
$R^3$ est l'hydrogène ou un groupe cyano,
$R^4$ est un halogène, et
$R^5$ est l'hydrogène ou un halogène.

2. Procédé de production des composés nouveaux de formule I

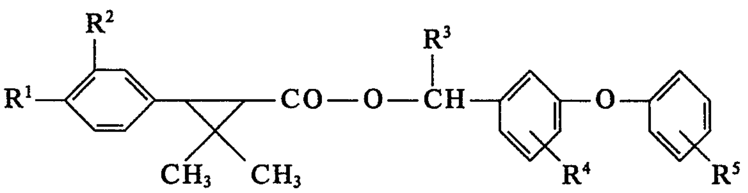

(I)

dans laquelle

$R^1$ représente des restes alkoxy ou alkylthio portant un ou plusieurs substituants halogéno égaux ou différents,
$R^2$ est l'hydrogène ou un halogène,
$R^1$ et $R^2$ forment ensemble un groupe alkylènedioxy portant un ou plusieurs substituants halogéno égaux ou différents,
$R^3$ est l'hydrogène ou un groupe cyano,
$R^4$ est un halogène, et
$R^5$ est l'hydrogène ou un halogène,

caractérisé en ce qu'on fait réagir des acides 2,2-diméthyl-3-phénylcyclopropanecarboxyliques substitués de formule II

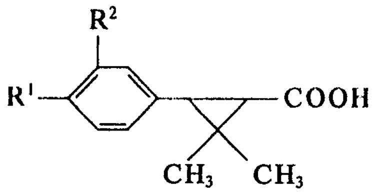

(II)

dans laquelle

$R^1$ et $R^2$ ont les définitions indiquées ci-dessus

— ou leurs dérivés réactifs, avec des alcools 3-phénoxybenzyliques de formule III

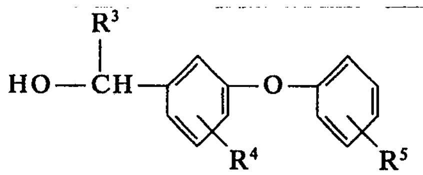

dans laquelle

$R^3$, $R^4$, $R^5$ ont les définitions indiquées ci-dessus,

ou avec leurs dérivés réactifs, éventuellement en présence d'un accepteur d'acide et/ou d'un catalyseur et, le cas échéant, en utilisant un ou plusieurs diluants.

3. Acides 2,2-diméthyl-3-phénylcyclopropane-carboxyliques substitués de formule II

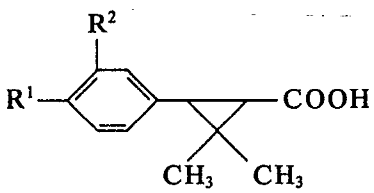

(II)

dans laquelle

$R^1$ représente des restes alkoxy en $C_1$ à $C_4$ ou alkylthio en $C_1$ à $C_4$ portant un ou plusieurs substituants halogéno égaux ou différents,

$R^2$ est l'hydrogène ou un halogène, ou bien

$R^1$ et $R^2$ forment ensemble un reste alkylènedioxy portant un ou plusieurs substituants halogéne égaux ou différents.

4. Procédé de production des nouveaux composés de formule (II) suivant la revendication 3, caractérisé en ce qu'on chauffe des esters d'acides 2,2-diméthyl-3-phénylcyclopropanecarboxyliques substitués de formule (IV)

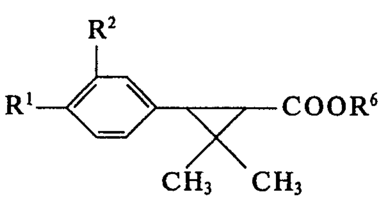

(IV)

dans laquelle

$R^1$ et $R^2$ ont les définitions indiquées ci-dessus et

$R^6$ est un groupe alkyle en $C_1$ à $C_4$,

avec des hydroxydes alcalins en présence de diluants à des températures comprises entre 50 et 200° C, puis on acidifie avec des acides minéraux à la température ambiante.

5. Esters d'acides phénylcyclopropanecarboxyliques substitués de formule (I) suivant la revendication 1, caractérisés en ce que

$R^1$ représente un groupe fluoralkoxy en $C_1$ ou $C_2$, chlorofluoralkoxy en $C_1$ ou $C_2$, fluoralkylthio en $C_1$ ou $C_2$ ou chlorofluoralkylthio en $C_1$ ou $C_2$,
$R^2$ est l'hydrogène, le chlore ou le brome,
$R^1$ et $R^2$ forment ensemble un groupe fluoralkylènedioxy en $C_1$ ou $C_2$,
$R^3$ représente l'hydrogène ou le groupe cyano,
$R^4$ est le fluor, le chlore ou le brome, et
$R^5$ est l'hydrogène, le fluor, le chlore ou le brome.

6. Esters d'acides phénylcyclopropanecarboxyliques substitués de formule (I) suivant la revendication 1, caractérisés en ce que

$R^1$ est un groupe chlorotrifluoréthoxy, tétrafluoréthoxy, chlorodifluorométhoxy, trifluorméthoxy, trifluorométhylthio et difluorométhoxy,
$R^2$ est l'hydrogène, le chlore ou le brome,
$R^1$ et $R^2$ forment conjointement un groupe trifluoréthylènedioxy ou difluorométhylènedioxy,
$R^3$ est l'hydrogène ou le groupe cyano,
$R^4$ est un radical 4-fluoro, 4-chloro ou 4-bromo, et
$R^5$ représente l'hydrogène.

7. Composés pesticides, caractérisés par une teneur en au moins un ester d'acide phénylcyclopropanecarboxylique substitué de formule I.

8. Utilisation d'esters d'acides phénylcyclopropanecarboxyliques substitués de formule (I) pour combattre des parasites.